# EUROPEAN PATENT SPECIFICATION

(11) **EP 3 801 419 B1**
(45) Date of publication and mention of the grant of the patent: **09.08.2023**
(21) Application number: 19758895.7
(22) Date of filing: 03.06.2019
(51) Int. Cl.: A61F 13/15, G01N 21/00, G06T 7/00

(54) **SUPERABSORBENT POLYMER APPLICATION AND CONTROL SYSTEM**
VORRICHTUNG ZUM AUFTRAGEN UND KONTROLLIEREN VON SUPERABSORBIERENDEN POLYMEREN
APPLICATION DE POLYMÈRE SUPERABSORBANT ET SYSTÈME DE COMMANDE

(30) Priority: 01.06.2018 GB 201809052
(43) Date of publication of application: 14.04.2021
(73) Proprietor: Cellulose Converting Solutions S.p.A., 65013 Citta Sant'Angelo (PE) (IT)
(72) Inventor: CIMINI, Carmine, 65129 Pescara Pescara (IT); RONCA, Giuseppe, 65129 Pescara Pescara (IT); SANTAVENERE, Alessandro, 66023 Francavilla Al Mare Chieti (IT)
(74) Representative: Plischke, Manfred
(86) International application number: PCT/EP2019/064388
(87) International publication number: WO 2019/229273

(56) References cited:
- US-A1- 2018 061 038

## Description

### Field of the Invention

The present invention relates to a radio frequency based image control system in the manufacturing of absorbent structures for absorbent articles, such as baby diapers, adult incontinence articles, but also bedpads, meat pads or the like. In particular, the present invention relates to a control system for the application of particulate material, especially superabsorbent material, which is adapted to function also for high speed manufacturing speeds.

### Background of the invention

Contact free control systems in the manufacture of absorbent articles are well known in the art, see e.g. EP2726039, wherein the positioning of various elements of an article are controlled by an image control system.

However, such a system can only view the outer contours of the article, such as fastening tapes. There is still a need for an in-line control system that also analyses "inner" parameter of the articles at a high speed, especially of the absorbent system, and more particular the amount and distribution of superabsorbent polymers (SAP).

WO2018/039535 (Accusentry) describes a method and an apparatus for simultaneously measuring and profiling in real-time the weight and the distribution of the fluff pulp and SAP during the production of absorbent articles. To this end, a video analysis system, operating at a wave length of between approximately 180 nm and approximately 2500 nm is combined with a radio frequency (RF) analysis system operating between 3 kHz and 3 GHz, though a range of from about 3 to 30 MHz appears to be highly preferred. The video analysis system provides data about the amount of fluff in the analyzed sample, whilst the RF analysis system provides data about the amount of SAP and fluff in the analyzed sample, such that by subtraction the amount of SAP can be determined.

In WO2018/204724 (Alcatera, published after the priority date of the present application), an RF analysis system is described for detecting the amount of SAP in an SAP containing absorbent structure, which operates at radio frequencies of between about 30 GHz and 10 THz, more preferably between about 300 GHz and 1 THz. In this range, the RF signal detects the amount of SAP material directly.

However, there is still a need for setting up a simple manufacturing system for the accurate and effective production of absorbent structures comprising SAP material with regard to amount, optionally of two different types of superabsorbent, if present, and positioning, both in machine and cross-machine direction.

### Summary of the Invention

In a first aspect, the present invention is a method for the continuous manufacture of absorbent structures comprising at least one x-y-directionally extending region comprising SAP on a high speed manufacturing equipment,
the method comprising the steps of
a) providing a manufacturing equipment comprising
   ∘ a carrier supply and support system
      operating at a x-directional line speed,
   ∘ a SAP supply system
   ∘ a SAP detection system
      comprising at least one electromagnetic wave (EM) radio frequency (RF) emitting emitter and at least one electromagnetic wave receiving detector, whereby each detector defines a detector resolution pixel of a predetermined size,
      optionally a multiplicity of emitters and/or detectors arranged in a linear or two-dimensional array,
      the detectors being adapted to provide a signal corresponding to the amount of SAP for each of the pixel regions of the absorbent structure;
   ∘ an encoder for generating a sequence of pulses serving as a speed related
      time reference signal;
   ∘ a control system for
      ▪ adjusting the SAP supply system or
      ▪ expelling selected absorbent structures
b) providing a predetermined target pattern of SAP distribution and / or a target amount of SAP per absorbent structure;
c) providing a carrier at a x-directional line speed
   ∘ preferably an essentially continuous web material
      optionally comprising cellulosic material;
d) adding SAP material by the SAP supply system to the carrier;
e) determining the actual pattern and actual amount by analysing the signal of the SAP detection system;
f) determining the deviation of the actual pattern and / or amount from the target pattern and / or amount for predetermined SAP comprising regions of the absorbent structures, for one or more parameter(s) selected from the group consisting of
   a. MD positioning of a single or multiple SAP comprising region(s);
   b. CD positioning of a single or multiple SAP comprising region(s); and
   c. amount of SAP of the regions of the absorbent structure preferably by a data processing system;
g) comparing the deviation with a first or second predetermined tolerance pattern / amount;
h) generating a signal for
   a. actuating process parameter adaptation, in case that the deviation from the tolerance pattern exceeds a first threshold; or
   b. actuating an expelling step, in case that the deviation from the tolerance exceed a second threshold.

The method may further satisfy one or more of the conditions selected from the group consisting of
a) the x-directional line speed being more than 20 m/ min, or preferably more than 100 m/min, or 300 m/min, or 500 m/min;
b) the absorbent structure production rate being more than 50 pieces / min, preferably more than 100 pieces / min, or more than 300 pieces / min, or more than 500 pieces / min or even over 1000 pieces / minute;
c) the SAP local basis weight averaged over a pixel size of the detector being more than about 20 g/m², more than 50 g/m², more than 100 g/m², more than 200 g/m², more than 300 g/m² or even more than 600 g/m²;
d) the absorbent structure comprising a total amount of SAP material of more than 0.1 g/ piece, or more than 1 g/ piece, or more than 3 g/ piece, or more than 5 g/ piece, or more than 8 g/ piece, or more than 10 g/ piece, or more than 12 g/ piece or more than 15 g/ piece or more than 18 g/ piece, or more than 20 g/ piece.

Optionally, multiple types of SAP may be added in overlaying patterns.

The detection system comprises an emitter for emitting electromagnetic waves in the range of more than 100, or more than 200 GHz and less than 900 or less than 500 GHz. The method may further comprise a product change system for adjusting the target pattern and/or the target amount of SAP and the SAP supply system setting cooperatively, preferably adapted for an "on the fly" product change.

The method may suitably be employed for the manufacture of a series of absorbent structures on a high speed manufacturing equipment, and preferably further comprise
- determining the deviation pattern of a predetermined number of subsequent articles;
- determining an average deviation pattern for the predetermined number of the subsequent articles;
- comparing the average deviation pattern with a tolerance pattern;
   determining a correction pattern signal to the control system; or
- adjusting the set points of the control system to reduce, preferably eliminate, the deviation of the deviation pattern to the target patter of a further sequence of subsequent articles.

Even further, the method may further comprise the steps of
- providing an adjustment tracking system;
- comparing the occurrence of adjustments to pre-set adjustment limits;
- inducing an action based on the comparison, the action being selected from the group consisting of
   - providing an alarm signal to an operator;
   - expelling a series of articles;
   - stopping the manufacturing operation,
- and optionally further comprise the step of adjusting the target pattern and or amount of SAP for executing a product type change, preferably an "on the fly" product change.

In a further aspect, the present invention is a method for the manufacture of absorbent articles comprising such absorbent structures

In a further aspect, the present invention is an equipment for the manufacture of absorbent articles comprising
- a carrier supply and support system operating at a x-directional line speed,
- a SAP supply system, and
- a SAP detection system
   comprising at least one electromagnetic wave (EM) radio frequency (RF) emitting emitter and at least one electromagnetic wave receiving detector
   adapted for being operated electromagnetic waves in the range of more than 100,
   or more than 200 GHz and less than 900 or less than 500 GHz,
   whereby each detector defines a detector resolution pixel of a predetermined size, optionally a multiplicity of emitters and/or detectors arranged in a linear or two-dimensional array, whereby the detectors are adapted to provide a signal corresponding to the amount of SAP for each of the pixel regions of the absorbent structure;
- an encoder for generating a sequence of pulses serving as a speed related time reference signal;
- a control system for
   ∘ adjusting the SAP supply system or
   ∘ expelling selected absorbent structures

In yet a further aspect, the present invention is a computer implemented control method in a continuous manufacturing system for a series of absorbent structures operating on a continuous web comprising SAP material, comprising the steps of
A) receiving in a data processing system comprising
   a. RF signals from an EM detection system, and
   b. a trigger signal from the encoder system adapted to identify individual absorbent structures;
B) generating a RF profile in the data processing system for an individual absorbent structure in the data processing system;
C) calculating in the data processing system an actual SAP weight profile for the individual absorbent structure;
D) comparing the actual SAP weight profile of an individual absorbent structure with a pre-set target profile in the data processing system;
E) calculating with a pre-set algorithm in the data processing system control signals for the SAP dosing and positioning systems;
F) sending the control signal of step E) from the data processing system to the SAP dosing and positioning systems;
G) actuating the SAP dosing and positioning systems by PLC(s) upon receipt of the control signals of step E and F);
and repeating steps A to G) for subsequent absorbent structures.

### Brief description of the drawings

Fig. 1 A to E depict schematically absorbent structures and articles, as may be suitably be produced by the methods or through the equipment according to the present invention.
Fig. 2A to C depict schematically executions of a manufacturing system and its operation according to the present invention.
Fig. 3 A to C depict absorbent structures when analysed by a detection system suitable for the present invention.

Same numerals in the figures refer to same or equivalent elements or features.

### Detailed Description

In a first aspect, the present invention is a method for manufacturing absorbent structures, as may be absorbent cores, as well as to absorbent articles comprising such structures or cores, such as feminine napkins, panty liners, diapers, diaper pants, adult incontinence, bed sheets, puppy pads, and other products incorporating the use of absorbent structures or cores. In a second aspect, the present invention is a system for manufacturing such absorbent structures, cores or articles. In a third aspect, the present invention is computer implemented method of controlling a manufacturing process for such absorbent structures, cores or absorbent articles. The absorbent structures comprise superabsorbent polymers, hereinafter also referred to as "SAP", such as well-known to a person skilled in the art of disposable absorbent articles. The absorbent articles may essentially comprise only an absorbent structure or core, such as in the case of absorbent pads such as bed pads, where the absorbent material may be enveloped between a liquid permeable topsheet and a liquid impermeable backsheet, or the absorbent articles may comprise other features, such as gasketing or closure features as well known for baby or adult incontinence diapers. Also, the absorbent core may be composed of sub-structures, such as a liquid acquisition and distribution system and a liquid storage system, at least the latter thereof comprising SAP. As will be discussed in more detail herein below, it is an important aspect of the present invention that the manufacturing system comprises or the methods utilize a particular detection system that applies electro-magnetic sensing technology with an emitter for emitting electromagnetic waves in the range of more than 100, preferably more than 200 GHz or more than 300 GHz, but less than 900 GHz, preferably less than 600 GHz, hereinafter referred to as "EM" (for electromagnetic) or "RF" (for radio frequency). Suitable systems have been developed e.g. by Alcatera LLC, Los Angeles, USA, or Terasense Group Inc. USA.

Articles according to the present invention may exhibit a wide range of dimensions such as an article width that may be as low as 1 cm or even less, or less than about 10 cm or less than about 20 cm, or less than about 30 cm, or less than about 50 cm. For even larger articles, such as bedpads, the width may be as wide as 100 cm or even more. Absorbent structures or cores of such articles are typically slightly smaller than the articles, such that there may be an absorbent free perimeter around the absorbent core to enable sealing that may be as small as 5 mm, often be around 10 mm, but reaching up to 50 mm, whereby the perimeter width may vary around the article.

It is also contemplated that two or more articles may be positioned cross-directionally adjacently to each other, either in a staggered arrangement, e.g. for so called "dog-bone" shaped articles as well known for feminine hygiene articles, but also in a non-staggered arrangement, as may be more suitable for baby or adult incontinence articles.

Often, though not necessarily, the length of the article exceeds the width thereof.

The length may be as low as 20 cm or even less, or less than about 10 cm or less than about 20 cm, or less than about 30 cm, or less than about 50 cm. For even larger articles, such as bedpads, the length may be as long as 100 cm or even more.

An absorbent structure for such articles may comprise low amounts of SAP material as may be well expressed by the basis weight, which may be as low as 20 g/m², but could be more than 50 g/m², more than 100 g/m², more than 200 g/m², more than 300 g/m² or even more than 600 g/m². Typically, the basis weight will be less than about 2000 g/m². The SAP material basis weight may be averaged for a total absorbent structure, or it may be related to a sub-region of the absorbent structure, such as of the pixel size of the detector, as described herein below.

Often, the SAP material is in a particulate form, typically with a particle size of more than about 45 µm and less than about 2000 µm, often less than 1200µm or less than about 800 µm. However, the SAP material may also be in other forms, such as a fiber, such as available from Technical Absorbents, UK, or even in foamed form.

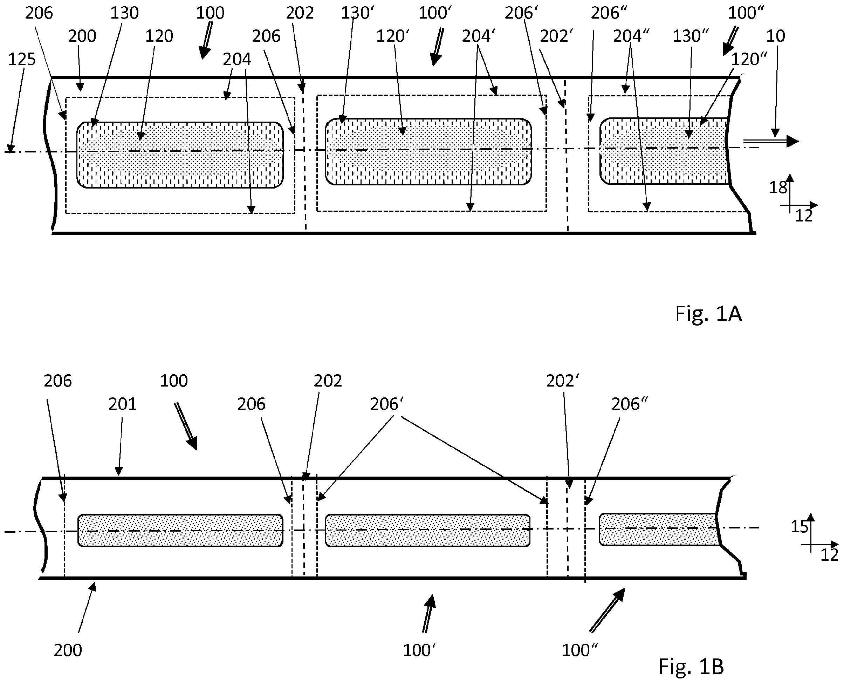

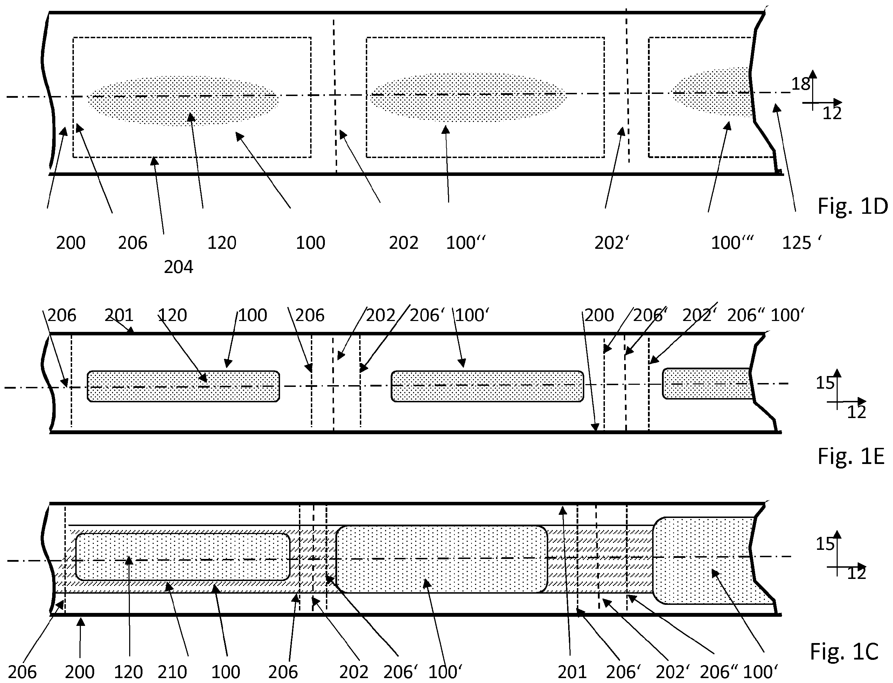

In Fig. 1A, several exemplary executions for absorbent structures are schematically depicted, as may be absorbent articles, such as absorbent pads, or absorbent structures or cores for such articles in a top view with a length or x-direction 12 of the structures, corresponding to the machine direction of a manufacturing process and a width, y-, or cross-direction 18. Fig. 1A depicts a sequence of absorbent structures 100, 100', 100" sandwiched between continuous webs, whereby only the supporting or carrier web 200 is shown, whilst the cover web is not shown in Fig. 1A. The web comprises separation lines 202, 202', as may be imaginary at that process stage, indicating the separation of structures (or cores or articles) at a later stage in the process to where the web is moving at a machine directional web speed 10. Both sandwiching webs may be liquid permeable, e.g. tissues or hydrophilic or hydrophilized nonwoven webs, e.g. for absorbent cores, or one of the webs may be liquid impermeable, such as when the structure forms an absorbent article, such as a bed pad. In Fig. 1A, the absorbency maybe delivered by a combination of fibrous material 130, e.g. fluff or pulp, and SAP, e.g. particulate SAP 120. Optionally, the structure may comprise sealing elements to contain absorbent material, such as x-directionally extending sealing lines 204, 204', ... and y-directionally extending sealing lines 206, 206', .... Fig. 1B depicts schematically a cross-directional view with machine or x-direction 12 and thickness or z-direction 15, with the absorbent structures 100, 100', 100" sandwiched between continuous webs 200 and 201, separation lines 202, and CD-sealing lines 206. An absorbent structure may comprise the SAP material homogeneously or inhomogeneously intermixed with other material, such as natural fibers, such as cellulosic pulp, or synthetic material, such as man-made fibers, such as olefin based fibers, or polyester baser fibers, or foamed materials. Fig. 1C depicts schematically a cross-sectional view of an absorbent structure comprising an open pore nonwoven web 210 with SAP particles embedded therein. The SAP particle may be completely within the pores of the nonwoven web 210, or partly be within the structure and partly on the surface of the structure.

The SAP may also be positioned in a layered form, such as when SAP particles 120 are placed on and sandwiched between a carrier 200 and cover 201 web material, such as a non-woven material or a paper tissue. In such a layer, the SAP may be in continuous layer or - as often preferred - in a patterned arrangement. The latter is a typical set up when individual articles are produced from the absorbent structures with an SAP material free zone machine directionally positioned between two adjacent absorbent structures. Preferably, at least the separation lines 202, preferably also the sealing lines 204 and 206 are in such material free zones, as indicated in Fig. 1D and E. However, also within one absorbent structure, the SAP material may be positioned in an x-y-directionally patterned arrangement. Also, the absorbent structure may comprise z-directionally a gradient of SAP material, such as a continuous gradient, wherein an upper portion along the z-direction comprises more SAP material per unit volume, and a lower portion comprises less, or vice versa. The z-directional gradient may also be achieved by layering SAP material, optionally separated by web materials or a wad of fibers, whereby some or more of the SAP material may also penetrate into the web or wad. Optionally one or more of these layers may be patterned x-y-directionally, as described in the above.

Furthermore, the absorbent structure may comprise different types of SAP materials exhibiting a different spectral attenuation, that may be placed in different layers, different portions of an x-y patterns, or be distributed inhomogeneously in a wad of fibers.

In Fig. 1, the positioning of SAP in subsequent individual absorbent structures is indicated as including some variation be it with regard to distance from the longitudinal centreline 215, or the MD- or CD extension of the SAP, the MD- or CD-positioning of the SAP, or overall or local SAP weight or weight distribution - all of which may be due to typical process variation.

A "target" SAP distribution pattern reflects the desired distribution of the SAP material in an absorbent structure or articles comprising such structures.

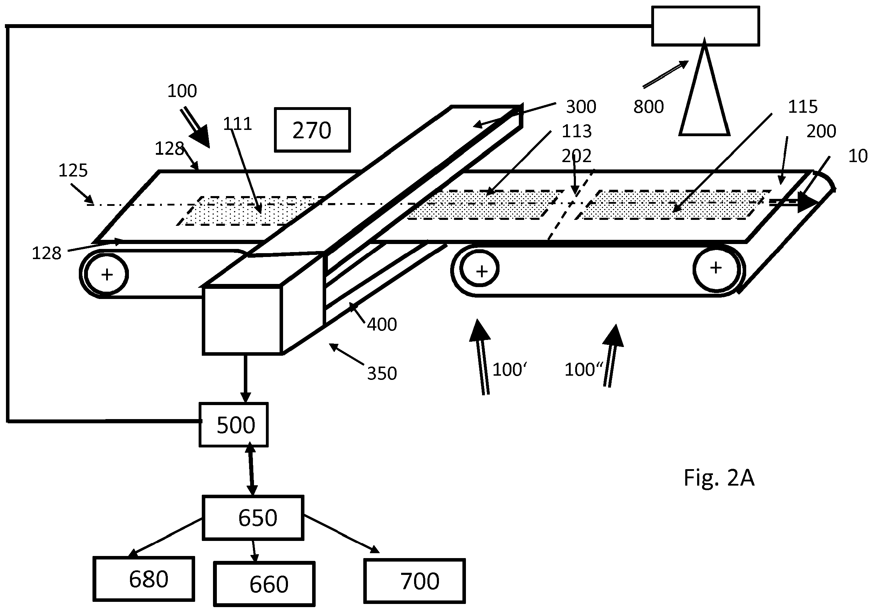

The key elements for a suitable manufacturing system for the manufacturing of such absorbent structures and the method of operating such a system is now further explained by referring to Fig. 2A to C.

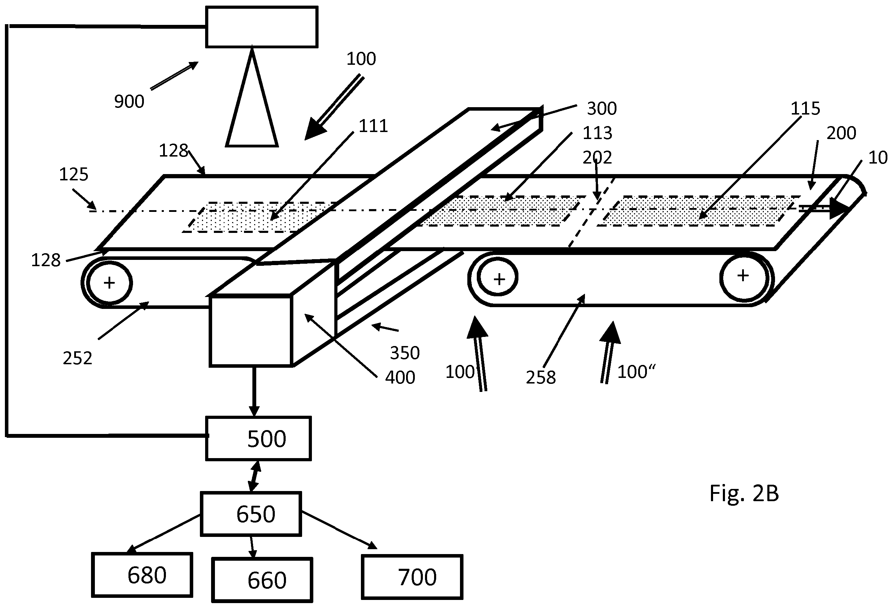

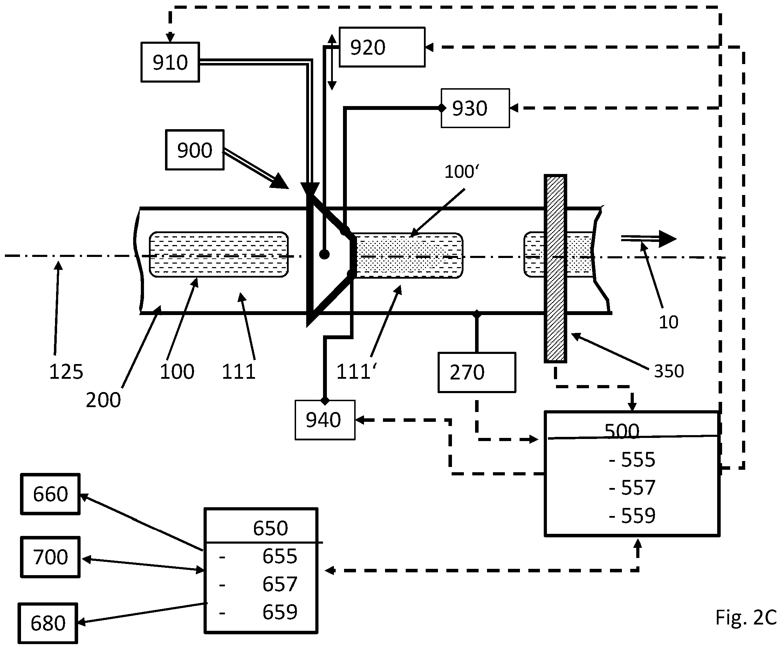

Typically, such a system comprises a carrier supply and support system comprising a carrier supply (not shown) and transfer elements, here shown as transfer belts 252 and 258, on which a continuous carrier web 200 is travelling at a web speed 10 along a machine direction 12, aligned with the length or x-direction of the absorbent structure 100 positioned on the web, optionally covered by a cover web (not shown in Fig. 2). Most preferably, such a system may be operated at high production web speeds of 20 m/ min, or more than 100 m/min, or more than 300 m/min, preferably more than 500 m/min, at structure core or article production rates of more than 300 pieces / min, often more than 500 pieces / min or even over 1000 pieces / minute. Further, an encoder 270 is connected to the carrier supply and support system directly or via the web speed control system, which generates a sequence of pulses serving as a speed related time reference signal for triggering analysis of the signals, thusly enabling to detect individual absorbent structures in the sequence thereof.

Exemplarily shown is a series of absorbent structures 100', 100', 100'", here shown positioned on the continuous web 200 moving along machine direction 12 at a web speed 10 and comprising separation lines 202 (only two thereof shown). The absorbent structures comprise SAP comprising regions 111, 113, 115, as may exhibit - in analogy to the various absorbent structures as depicted in Fig 1 - varying positioning, dimensions, or amount of SAP resulting from process variations in the SAP supply system or the web supply and support system.

The system further comprises a detection system 350, comprising an EM / RF emitter system 300 and a receiver system 400 operating at a range of more than 100, preferably more than 200 GHz, but less than 900 GHz, preferably less than 600 GHz, most preferably in the range of between 300 GHz and 500 GHz.

An EM emitter system 300 directs EM rays through the absorbent structures 100', 100", 100‴ towards a sensor system 400, therein creating an electric signal as may be further converted by a first data processing system 500, including a signal transformation means.

The detection system comprises an EM wave emitter system that emits EM waves preferably along the z-direction of the machine, which is aligned with the length direction of the article. Thus the emitting system comprises at least one essentially punctiform emitter of a typical diameter of typically less than 10 mm, or less than 5 mm or even smaller, or a linear or two-dimensional array of such punctiform emitters.

Preferably, the intensity is essentially constant over time, or can be adjusted.

The detection system further comprises a receiver system or at least one, preferably a multiplicity of detectors that are adapted to receive the emitted EM waves and to create a signal of the EM modulated by the amount and/or type of SAP material positioned between the emitter and the receive. In a preferred execution, the receivers arc linearly arranged, or on a patterned array with several lines, that optionally may be offset cross-directionally as may provide a better y-directional resolution. The sensor detecting system should be able to capture the EM pictures of the absorbent structures at a high sampling rate, preferably of 24 kHz or more. Suitable detection systems are disclosed in the above referenced publication WO2018/204724 (Alcatera), similar systems are manufactured by Terasense Group Inc. San Jose, CA, USA.

Each of the detectors has a predetermined pixel resolution size that is less than about 10 mm by 10 mm, preferably better than 5 mm by 5 mm, more preferably less than 2 mm by 2 mm.

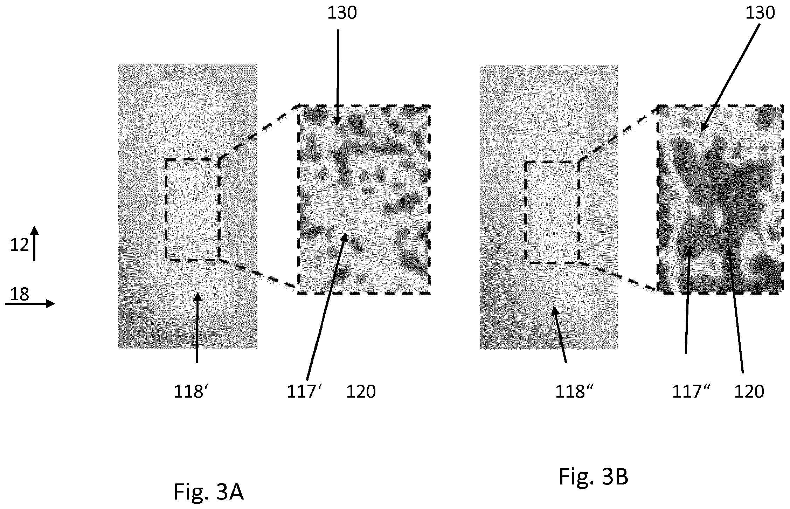

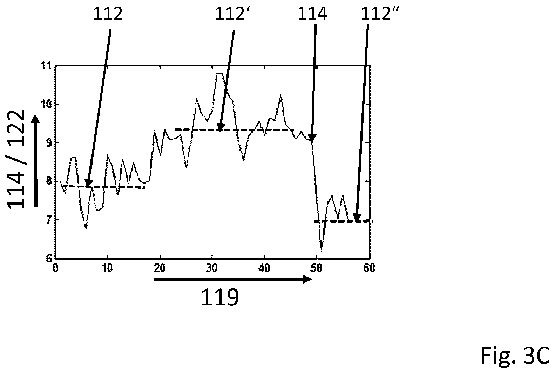

As schematically and exemplarily, but non-limiting presented in Fig. 3A and B, showing an absorbent article 118' with an absorbent structure (Fig. 3A) and 118" (Fig. 3B) in a visible light photographic picture without any discernible difference. The enlarged sections 117' and 117", respectively, thereof, viewed by an EM sensor/detection system, depict the SAP material 120 as darker areas as embedded in pulp 130. The articles 118 exhibit an extension in the x- or length direction 12 and an extension in the y- or width direction, with the height, thickness or z-direction not shown in the figure. The system reflects the local amount, or z-directional distribution, quantitatively, i.e. the darker a certain region is shown, the higher is the local amount of SAP. Further, the system depicts the x-y-directional distribution, both with regard to distribution evenness and positioning. Further, as exemplarily depicted in Fig. 3C, showing the actual curve (114) and target plateaus (112, 112' and 112") for the amount of SAP, e.g. of an article or an absorbent structure for an article, over a series of articles 119.

The detection system further comprises a system for transforming the signal of the receiver in to a pattern for the actual SAP distribution in an absorbent structure, as may be compared to the target pattern of the absorbent structure or article. The system may then be operated in two non-excluding, set-ups and operation modes.

In a first mode as depicted in Fig. 2A, the signal is compared by a computational system or data processing system 500 to the preset target pattern as may be provided from a further data processing system 650, which may be further connected to a Graphical User Interface (GUI) 660, to a further data storage unit 680 or to a Human-Machine-Interface (HMI) 700. The further data processing system may serve to input process parameter such as the target pattern, on which the computational system 500 signals to an expelling unit 800 to remove defect articles from the manufacturing line, and/or providing a warning signal to an operator. Such a system allows very good quality control, but may create significant amount of scrap articles.

In a second, non-exclusive mode, as depicted generally in Fig. 2B, the signal is also compared in the computational system 500, however a signal is fed to an SAP feeding system 900 whereby the SAP feeding may be corrected in-line and automatically by a data processing system without the need for action by an operator.

Fig. 2C depicts in more detail the computer implemented method to control the system. To this end, a data processing system 500 comprises a data processor unit 555, a programming unit 557 and a data storage or memory unit 559. The data processing system 500 is connected to
- an EM detection system 350 comprising the RF emitter 300 and sensor system 400;
- an encoder 270;
- an SAP feeding system 900, comprising
   ∘ a SAP supply
   ∘ a SAP dosing unit, such as screw feeder 910;
   ∘ one or more SAP feed positioning system(s), adapted to allow adjusting SAP distribution profile via
      ▪ MD positioning,
      ▪ CD positioning,
      ▪ width of a SAP curtain, if present,
      each element of the SAP feeding system comprising programmable logic controller (PLC) units for adjusting system parameter upon a received control signal;
- and a further data processing system 650 comprising a data processor unit 655, a programming unit 657, and a data storage or memory unit 659, as may further be connected to a Graphical User Interface (GUI) 660, to a further data storage unit 680 or to a Human-Machine-Interface (HMI) 700;

The dosing unit as well as the positioning systems may comprise stepper motors, pneumatics positioners, or flow diverters. Preferably they comprise closed loop servo motors, exhibiting high dynamics, low response time, preferably of less than 10 ms, and low positioning error. Optionally, they may operate according to pre-set and programmable movement or velocity profiles. Preferably, the PLC units apply a proportional-integral-derivative (PID) control logic, optionally as cascading PID control, e.g. a first level PID controlling deviations resulting from the overall process speed, a second level controlling feed or positioning deviations.

The encoder allows triggering the analysis system so as to enable separated analysis of subsequent absorbent structures. The encoder produces a sequence of pulses that serve to clock the acquisition of data into equal-spaced slices of the sample. The numbers of encoder pulses per meter of product displacement are related to the pixel dimensions, i.e. the scanner resolution. Small Pixel are useful for check distribution of SAP. The total amount can be calculated also with "one single pixel" (one line of a signal receiving unit) in cross direction of product movement.

Preferably, in order to minimize the dead time between the data generation and the system reacting thereon, the positioning of the detector system 350 is a close as possible to the formation of the absorbent structure, preferably at a distance along the machine direction of less than 20, preferably less than 10, even more preferably less than 5 times the unit length of an absorbent structure.

Thus, the computer implemented control method in a continuous manufacturing system for absorbent structures operating on a continuous web comprising SAP material, comprises the steps of
A) receiving in a data processing system 500
   a. RF signals from an EM detection system 350, and
   b. a trigger signal from the encoder system 270;
B) generating a combined RF profile in the data processing system 500 for a single absorbent structure in said data processing system 500;
C) calculating in the data processing system 500 an actual SAP weight profile for the single absorbent structure;
D) comparing the actual SAP weight profile with a pre-set target profile in the data processing system 500;
E) calculating with a pre-set algorithm in the data processing system 500 control signals for the SAP dosing and positioning systems;
F) sending the control signal of step E) from the data processing system 500 to the SAP dosing and positioning systems 910, 920, 930, or 940;
G) actuating the SAP dosing and positioning systems by the PLC(s) upon receipt of the control signals of step E and F);
H) repeating steps A to G) for subsequent absorbent structures.

The data processing system 500 may be connected to a further data processing system 650, such that the further data processing system 650 provides the targets for the data processing systems, e.g. for the SAP distribution pattern, or for other process control parameter. Also, the first data processing system 500 may submit the control signals to the further processing system 650, where these may be used for further automated analysis by algorithms providing also automated parameter adaptation for the first data processing system, e.g. for establishing more stable process conditions and/or identifying process instabilities.

The computational system may also be adapted to allow quick, preferably "on-the-fly" changes from one article design to a different one, by providing different target patterns and switching from one to another.

The EM detector system may suitably be positioned in a gap between to web transport means, such as indicated in Fig. 3A and B as transfer belts 252, 258.

The skilled person will readily realize, that this system may also be combined with units for expelling or "rejecting" articles, but as the correction may already take place a small deviations as may also be averaged over a series of articles, and may be compared to specific tolerance patterns for particular parameter. For example, the positioning in x-direction may vary by a certain amount, e.g. 5 mm, whilst the deviation in cross-direction may only be 2 mm.

Whilst the system as depicted in the figures shows the detector and emitter in a laying U-shape with the web, respective the absorbent structures or articles being fed between them, it is contemplated, that the emitter(s) and detector(s) do not need to be in a common structure. Also, for example for webs or articles exhibiting a larger width, two or more units may be applied, e.g. one from each side longitudinally off-set.

Further, when considering the use of two or even more different types of SAP the SAP application system 900 may just be multiplied, and the data processing system may provide separate signal to each thereof.

## Claims

1. A method for the continuous manufacture of an absorbent structure comprising at least one x-y-directionally extending region comprising SAP on a high speed manufacturing equipment
said method comprising the steps of
- providing a manufacturing equipment comprising
∘ a carrier supply and support system
operating at a x-directional line speed,
∘ a SAP supply system
∘ a SAP detection system
comprising at least one electromagnetic wave (EM) radio frequency (RF) emitting emitter and at least one electromagnetic wave receiving detector, whereby each detector defines a detector resolution pixel of a predetermined size,
optionally a multiplicity of emitters and/or detectors arranged in a linear or two-dimensional array,
said detectors being adapted to provide a signal corresponding to the amount of SAP for each of said pixel regions of said absorbent structure;
o an encoder for generating a sequence of pulses serving as a speed related time reference signal;
∘ a control system for
▪ adjusting said SAP supply system or
▪ expelling selected absorbent structures
- providing a predetermined target pattern of SAP distribution and / or a target amount of SAP per absorbent structure;
- providing a carrier at a x-directional line speed
o preferably an essentially continuous web material
optionally comprising cellulosic material;
- adding SAP material by said SAP supply system to said carrier;
- determining the actual pattern and actual amount by analysing the signal of said SAP detection system;
- determining the deviation of said actual pattern and / or amount from said target pattern and / or amount for predetermined SAP comprising regions of said absorbent structures, for one or more parameter(s) selected from the group consisting of
o MD positioning of a single or multiple SAP comprising region(s);
o CD positioning of a single or multiple SAP comprising region(s); and
o amount of SAP of said regions of said absorbent structure
preferably by a data processing system;
- comparing said deviation with a first or second predetermined tolerance pattern / amount;
- generating a signal for actuating process parameter adaptation, in case that said deviation from said tolerance pattern exceeds a first threshold; or
o actuating an expelling step, in case that said deviation from said tolerance exceed a second threshold,
wherein said detection system comprises an emitter for emitting electromagnetic waves in the range of more than 100, or more than 200 GHz and less than 900 or less than 500 GHz.

2. A method according to claim 1, further satisfying one or more of the conditions selected from the group consisting of
a) said x-directional line speed being more than 20 m/ min, or preferably more than 100 m/min, or 300 m/min, or 500 m/min;
b) the absorbent structure production rate being more than 50 pieces / min, preferably more than 100 pieces / min, or more than 300 pieces / min, or more than 500 pieces / min or even over 1000 pieces / minute;
c) the SAP local basis weight averaged over a pixel size of the detector being more than about 20 g/m², more than 50 g/m², more than 100 g/m², more than 200 g/m², more than 300 g/m² or even more than 600 g/m²;
d) the absorbent structure comprising a total amount of SAP material of more than 0.1 g/ piece, or more than 1 g/ piece, or more than 3 g/ piece, or more than 5 g/ piece, or more than 8 g/ piece, or more than 10 g/ piece, or more than 12 g/ piece or more than 15 g/ piece or more than 18 g/ piece, or more than 20 g/ piece.

3. A method according to any of claims 1 to 2, comprising the addition of multiple types of SAP in overlaying patterns.

4. A method according to any of claims 1 to 3, further comprising a product change system for adjusting said target pattern and/or said target amount of SAP and said SAP supply system setting cooperatively, preferably adapted for an "on the fly" product change.

5. A method for the manufacture of a series of absorbent structures on a high speed manufacturing equipment,
said method comprising the manufacturing of a series of absorbent articles according to any of claims 1 to 4,
said method further comprising
- determining the deviation pattern of a predetermined number of subsequent articles;
- determining an average deviation pattern for said predetermined number of said subsequent articles;
- comparing said average deviation pattern with a tolerance pattern;
determining a correction pattern signal to said control system; or
- adjusting the set points of said control system to reduce, preferably eliminate, the deviation of said deviation pattern to said target patter of a further sequence of subsequent articles.

6. A method according to claim 5, further comprising
providing an adjustment tracking system;
comparing the occurrence of adjustments to pre-set adjustment limits inducing an action based on said comparison, said action being selected from the group consisting of
providing an alarm signal to an operator;
expelling a series of articles;
stopping the manufacturing operation,
optionally further comprising the step of adjusting said target pattern and or amount of SAP for executing a product type change, preferably an "on the fly" product change.

7. An equipment for the manufacture of absorbent articles comprising
o a carrier supply and support system
operating at a x-directional line speed,
o a SAP supply system
o a SAP detection system
comprising at least one electromagnetic wave (EM) radio frequency (RF) emitting emitter and at least one electromagnetic wave receiving detector adapted for being operated electromagnetic waves in the range of more than 100, or more than 200 GHz and less than 900 or less than 500 GHz,
whereby each detector defines a detector resolution pixel of a predetermined size, optionally a multiplicity of emitters and/or detectors arranged in a linear or two-dimensional array,
said detectors being adapted to provide a signal corresponding to the amount of SAP for each of said pixel regions of said absorbent structure;
∘ an encoder for generating a sequence of pulses serving as a speed related time reference signal;
∘ a control system for
▪ adjusting said SAP supply system or
▪ expelling selected absorbent structures

8. A computer implemented control method in a continuous manufacturing system for a series of absorbent structures operating on a continuous web comprising SAP material, comprising the steps of
A - receiving in a data processing system comprising
- RF signals from an EM detection system ,
wherein said detection system comprises an emitter for emitting electromagnetic waves in the range of more than 100, or more than 200 GHz and less than 900 or less than 500 GHz,
and
- a trigger signal from the encoder system adapted to identify individual absorbent structures;
B - generating a RF profile in the data processing system for an individual absorbent structure in said data processing system;
C - calculating in the data processing system an actual SAP weight profile for the individual absorbent structure;
D - comparing the actual SAP weight profile of an individual absorbent structure with a pre-set target profile in the data processing system;
E - calculating with a pre-set algorithm in the data processing system control signals for the SAP dosing and positioning systems;
F - sending the control signal of step E) from the data processing system to the SAP dosing and positioning systems;
G - actuating the SAP dosing and positioning systems by PLC(s) upon receipt of the control signals of step E and F);
H - and repeating steps A to G) for subsequent absorbent structures.

## Patentansprüche

1. Ein Verfahren zur kontinuierlichen Herstellung absorbierender Strukturen auf einer Hochgeschwindigkeitsproduktionanlage, die mindestens einen in x-y-Richtung verlaufenden Bereich umfassen, der SAP enthält,
wobei das Verfahren die folgenden Schritte enthält:
- Bereitstellung einer Produktionsanlage, beinhaltend
o ein Trägerelement und Unterstützungssystem
welches mit einer Liniengeschwindigkeit in x-Richtung läuft,
o ein SAP-Versorgungssystem
o ein SAP-Erkennungssystem
enthaltend mindestens einen Emitter, der hochfrequenz (RF) elektromagnetische Wellen (EM) aussendet,
und mindestens einen Detektor, der elektromagnetische Wellen empfängt, wobei jeder Detektor ein Detektorauflösungspixel einer vorbestimmten Größe definiert,
optional eine Vielzahl von Emittern und/oder Detektoren, die in einem linearen oder zweidimensionalen Array angeordnet sind,
wobei die Detektoren so ausgelegt sind, dass sie ein Signal liefern, das der SAP-Menge für jeden der Pixelbereiche der absorbierenden Struktur entspricht;
∘ einen Encoder zum Erzeugen einer Impulsfolge, die als geschwindigkeitsbezogenes Zeitreferenzsignal dient;
∘ ein Kontrollsystem für
▪ die Anpassung des SAP-Versorgungssystems oder
▪ dem Ausstoß ausgewählter absorbierender Strukturen;
- Bereitstellen eines vorgegebenen Zielmusters der SAP-Verteilung und/oder einer Zielmenge an SAP pro absorbierende Struktur;
- Bereitstellen eines Trägerelements mit einer Liniengeschwindigkeit in x-Richtung
∘ vorzugsweise ein im Wesentlichen kontinuierliches Bahnmaterial optional Zellulosematerial enthaltend;
- Hinzufügen von SAP-Material durch das SAP-Versorgungssystem zu dem Träger;
- Bestimmen des tatsächlichen Musters und der tatsächlichen Menge durch Analyse des Signals des SAP-Erkennungssystems;
- Bestimmen der Abweichung des tatsächlichen Musters und/oder der tatsächlichen Menge vom Zielmuster und/oder der Zielmenge für vorgegebene SAP-Bereiche der absorbierenden Strukturen, für einen oder mehrere Parameter, ausgewählt aus der Gruppe bestehend aus
o MD-Positionierung einer einzelnen oder mehrerer SAP umfassende Region(en);
o CD-Positionierung einer einzelnen oder mehrerer SAP umfassenden Region(en); und
o Menge an SAP in den genannten Bereichen der genannten absorbierenden Struktur,
vorzugsweise durch ein Datenverarbeitungssystem;
- Vergleichen der Abweichung mit einem ersten oder zweiten vorgegebenen Toleranzmuster oder -betrag;
- Erzeugen eines Signals zur Auslösung der Prozessparameteranpassung für den Fall, dass die Abweichung vom Toleranzmuster einen ersten Schwellenwert überschreitet; oder
o Initiieren eines Ausstoßschritts für den Fall, dass die Abweichung von der Toleranz einen zweiten Schwellenwert überschreitet,
wobei das Detektionssystem einen Emitter zum Emittieren elektromagnetischer Wellen im Bereich von mehr als 100 oder mehr als 200 GHz und weniger als 900 oder weniger als 500 GHz enthält.

2. Verfahren nach Anspruch 1, das außerdem eine oder mehrere der Bedingungen erfüllt, ausgewählt aus der Gruppe bestehend aus
a) die Geschwindigkeit der x-Richtung der Linie beträgt mehr als 20 ml/min oder vorzugsweise mehr als 100 m/min oder 300 m/min oder 500 m/min;
b) die Produktionsrate der absorbierenden Struktur beträgt mehr als 50 Stück/Minute, vorzugsweise mehr als 100 Stück/Minute, oder mehr als 300 Stück/Minute, oder mehr als 500 Stück/Minute oder sogar über 1000 Stück/Minute;
c) das über eine Pixelgröße des Detektors gemittelte lokale SAP-Flächengewicht beträgt mehr als etwa 20 g/m², mehr als 50 g/m², mehr als 100 g/m², mehr als 200 g/m², mehr als 300 g/m². m²oder sogar mehr als 600 g/m²;
d) die absorbierende Struktur enthält eine Gesamtmenge an SAP-Material von mehr als 0,1 g/Stück, oder mehr als 1 g/Stück, oder mehr als 3 g/Stück, oder mehr als 5 g/Stück, oder mehr als 8 g/Stück Stück, oder mehr als 10 g/Stück, oder mehr als 12 g/Stück oder mehr als 15 g/Stück oder mehr als 18 g/Stück, oder mehr als 20 g/Stück.

3. Verfahren nach einem der Ansprüche 1 bis 2, das das Hinzufügen mehrerer SAP-Typen in überlagernden Mustern enthält.

4. Verfahren nach einem der Ansprüche 1 bis 3, weiterhin umfassend ein Produktwechselsystem zum zusammenwirkenden Anpassen des Zielmusters und/oder der Zielmenge an SAP und der Einstellung des SAP-Versorgungssystems, vorzugsweise angepasst für einen "on-the-fly"-Produktwechsel.

5. Ein Verfahren zur Herstellung einer Reihe absorbierender Strukturen auf einer Hochgeschwindigkeitsfertigungsanlage,
wobei das Verfahren die Herstellung einer Reihe absorbierender Artikel nach einem der Ansprüche 1 bis 4 enthält,
und das Verfahren weiterhin folgende Schritte enthält:
- Bestimmen des Abweichungsmusters einer vorgegebenen Anzahl aufeinanderfolgender Artikel;
- Bestimmen eines durchschnittlichen Abweichungsmusters für die vorgegebene Anzahl der aufeinanderfolgenden Artikel;
- Vergleichen des durchschnittlichen Abweichungsmusters mit einem Toleranzmuster; Bestimmen eines Korrekturmustersignals für das Steuersystem; oder
- Anpassen der Sollwerte des Steuersystems, um die Abweichung des Abweichungsmusters vom Zielmuster einer weiteren Folge aufeinanderfolgender Artikel zu verringern, vorzugsweise zu beseitigen.

6. Verfahren nach Anspruch 5, weiterhin enthaltend
Bereitstellung eines Anpassungsverfolgungssystems;
Vergleichen des Auftretens von Anpassungen mit voreingestellten Anpassungsgrenzen
Auslösen einer Aktion basierend auf dem Vergleich, wobei die Aktion aus einer Gruppe ausgewählt wird, bestehend aus
Bereitstellen eines Alarmsignals für einen Bediener;
Ausschluss einer Artikelserie;
Stoppen des Produktionsbetriebs,
optional weiterhin den Schritt des Anpassens des Zielmusters und/oder der SAP-Menge zur Durchführung einer Produkttypänderung, vorzugsweise einer "on-the-fly"-Produktänderung.

7. Eine Vorrichtung zur Herstellung absorbierender Artikel, enthaltend
o ein Trägerversorgungs- und Unterstützungssystem, das mit einer Liniengeschwindigkeit in x-Richtung läuft;
o ein SAP-Versorgungssystem;
o ein SAP-Erkennungssystem;
enthaltend mindestens einen Sender, der hochfrequente (RF) elektromagnetische Wellen (EM) aussendet, und mindestens einen Detektor, der elektromagnetische Wellen empfängt und vorzugsweise für den Betrieb elektromagnetischer Wellen im Bereich von mehr als 100 oder mehr als 200 GHz und weniger als 900 oder weniger ausgelegt ist als 500 GHz,
wobei jeder Detektor ein Detektorauflösungspixel einer vorgegebenen Größe definiert, optional eine Vielzahl von Emittern und/oder Detektoren, die in einem linearen oder zweidimensionalen Array angeordnet sind,
wobei die Detektoren so ausgelegt sind, dass sie ein Signal liefern, das der SAP-Menge für jeden der Pixelbereiche der absorbierenden Struktur entspricht;
o einen Encoder zum Erzeugen einer Impulsfolge, die als geschwindigkeitsbezogenes Zeitreferenzsignal dient;
o ein Kontrollsystem für
▪ die Anpassung des SAP-Versorgungssystems oder
▪ den Ausstoß ausgewählter absorbierender Strukturen;

8. Ein computerimplementiertes Steuerverfahren in einem kontinuierlichen Herstellungssystem für eine Folge absorbierender Strukturen, die auf einer kontinuierlichen Bahn aus SAP-Material betrieben werden, folgende Schritte enthaltend:
A - in einem Datenverarbeitungssystem, Empfang von
- RF Signalen von einem EM-Erkennungssystem,
wobei das Detektionssystem einen Emitter zum Emittieren elektromagnetischer Wellen im Bereich von mehr als 100 oder mehr als 200 GHz und weniger als 900 oder weniger als 500 GHz enthält,
und
- einem Triggersignal vom Encodersystem, das dazu geeignet ist, einzelne absorbierende Strukturen zu identifizieren;
B - Erzeugen eines RF-Profils im Datenverarbeitungssystem für eine einzelne absorbierende Struktur im Datenverarbeitungssystem;
C - Berechnen eines tatsächlichen SAP-Gewichtsprofils für die einzelne absorbierende Struktur im Datenverarbeitungssystem;
D - Vergleichen des tatsächlichen SAP-Gewichtsprofils einer einzelnen absorbierenden Struktur mit einem voreingestellten Zielprofil im Datenverarbeitungssystem;
E - Berechnen mit einem im Datenverarbeitungssystem voreingestellten Algorithmus Steuersignale für die SAP-Dosier- und Positionierungssysteme;
F - Senden des Steuersignals aus Schritt E) von der Datenverarbeitungsanlage an die SAP-Dosier- und Positionierungssysteme;
G - Betätigen der SAP-Dosier- und Positionierungssysteme durch SPS(s) nach Empfang der Steuersignale von Schritt E und F);
H - und Wiederholen der Schritte A bis G) für nachfolgende absorbierende Strukturen.

## Revendications

1. Procédé de fabrication en continu de structures absorbantes comprenant au moins une région s'étendant dans la direction x-y comprenant du SAP à un équipement de fabrication à grande vitesse
ledit procédé comprenant les étapes de
- fournir un équipement de fabrication comprenant
o un système d'approvisionnement et d'accompagnement des porteurs fonctionnant à une vitesse de ligne dans la direction x,
o un système d'approvisionnement de SAP ;
o un système de détection de SAP
comprenant au moins un émetteur émettant des ondes électromagnétiques (EM) radiofréquence (RF) et au moins un détecteur recevant des ondes électromagnétiques,
dans lequel chaque détecteur définisse un pixel de résolution de détecteur d'une taille prédéterminée,
éventuellement une multiplicité d'émetteurs et/ou de détecteurs arrangés en un groupement linéaire ou bi-dimensionnel,
lesdits détecteurs étant adaptés pour fournir un signal correspondant à la quantité de SAP pour chacune desdites régions de pixel de ladite structure absorbante;
∘ un codeur pour générer une séquence d'impulsions servant de signal de référence de temps lié à la vitesse ;
∘ un système de contrôle pour
▪ régler ledit système d'approvisionnement de SAP ou
▪ expulsion des structures absorbantes sélectionnées ;
- fournir un motif cible prédéterminé de distribution de SAP et/ou une quantité cible de SAP par structure absorbante ;
- fournir un porteur à une vitesse de ligne dans la direction x
o de préférence un matériau en bande essentiellement continu
comprenant éventuellement un matériau cellulosique;
- ajouter du matériel SAP par ledit système d'approvisionnement de SAP audit support ;
- déterminer le motif réel et la quantité réelle en analysant le signal dudit système de détection de SAP ;
- déterminer l'écart dudit motif réel et/ou quantité par rapport audit motif cible et/ou quantité de SAP prédéterminé dans les régions desdites structures absorbantes, pour un ou plusieurs paramètre(s) sélectionné(s) dans le groupe consistant par
o positionnement le long du MD d'un seul ou plusieurs région(s) comprenant(s) SAP
o positionnement le long du CD d'un seul ou de plusieurs region(s) comprenant(s) SAP; et
o quantité de SAP dans desdites régions de ladite structure absorbante de préférence par un système informatique;
- comparer ledit écart avec un premier ou un deuxième motif de la gamme de tolérance prédéterminé;
- générer un signal pour actionner l'adaptation des paramètres de processus, dans le cas où ledit écart par rapport audit motif de tolérance dépasse un premier seuil ; or
- actionner une étape d'expulsion, dans le cas où ledit écart à ladite gamme de tolérance dépasse un deuxième seuil.
dans lequel ledit système de détection comprend un émetteur pour émettre des ondes électromagnétiques dans la étendue de plus de 100, ou plus de 200 GHz et moins de 900 ou moins de 500 GHz.

2. Procédé selon la revendication 1, satisfaisant en outre une ou plusieurs des conditions choisies dans le groupe consistant par
a) ladite vitesse de ligne dans la direction x étant supérieure à 20 m/ min, ou de préférence supérieure à 100 m/min, ou 300 m/min, ou 500 m/min ;
b) la vitesse de production de la structure absorbante étant supérieure à 50 pièces/min, de préférence supérieure à 100 pièces/min, encore plus que 300 pièces/min, ou encore plus que500 pièces/min, ouencore plus que 1000 pièces/minute;
c) le grammage local SAP moyenné sur une taille de pixel du détecteur étant supérieur à environ 20 g/m², supérieur à 50 g/m², ou supérieur à 100 g/m², ou supérieur à 200 g/m², ou supérieur à 300 g/ m² ou même plus de 600 g/m² ;
d) la structure absorbante comprenant une quantité totale de matériau SAP supérieure à 0,1 g/pièce, ou supérieure à 1 g/pièce, ou supérieure à 3 g/pièce, ou supérieure à 5 g/pièce, ou supérieure à 8 g/pièce pièce, ou plus de 10 g/pièce, ou plus de 12 g/pièce ou plus de 15 g/pièce ou plus de 18 g/pièce, ou plus de 20 g/pièce.

3. Procédé selon l'une quelconque des revendications 1 à 2, comprenant l'ajout de multiples types de SAP dans des motifs superposés.

4. Procédé selon l'une quelconque des revendications 1 à 3, comprenant en outre un système de changement de produit pour ajuster ledit motif cible et/ou ladite quantité de SAP cible et ledit réglage du système d'alimentation SAP de manière coopérative, de préférence adapté pour un changement de produit "à la volée".

5. Procédé de fabrication d'une série de structures absorbantes sur un équipement de fabrication à grande vitesse,
ledit procédé comprenant la fabrication d'une série d'articles absorbants selon l'une quelconque des revendications 1 à 4,
ledit procédé comprenant en outre
- déterminer le motif d'écart d'un nombre prédéterminé d'articles suivants ;
- déterminer un motif d'écart moyen pour ledit nombre prédéterminé desdits articles suivants ;
- comparer ledit motif d'écart moyen avec un motif de tolérance; déterminer un signal de motif de correction vers ledit système de commande; ou
- ajuster les points de réglage dudit système de commande pour réduire, de préférence éliminer, l'écart dudit motif d'écart vers ledit motif cible d'une autre séquence d'articles suivants.

6. Procédé selon la revendication 5, comprenant en outre
fournir un système de suivi des ajustements ;
comparer l'occurrence des ajustements aux limites d'ajustement prédéfinies induire une action sur la base de ladite comparaison, ladite action étant sélectionnée dans le groupe consistant par
fournir un signal d'alarme à un opérateur ;
expulser une série d'articles;
arrêter l'opération de fabrication,
comprenant éventuellement en outre l'étape d'ajustement dudit motif cible et/ou de la quantité de SAP pour exécuter un changement de type de produit, de préférence un changement de produit "à la volée".

7. Equipement pour la fabrication d'articles absorbants comprenant
o un système d'approvisionnement et d'accompagnement des porteurs fonctionnant à une vitesse de ligne dans la direction x
o un système d'approvisionnement de SAP
o un système de détection de SAP
comprenant au moins un émetteur émettant des ondes électromagnétiques (EM) radiofréquence (RF) et au moins un détecteur récepteur d'ondes électromagnétiques de préférence adapté pour être actionné par des ondes électromagnétiques dans l'étendu de plus de 100, ou plus de 200 GHz et moins de 900 ou moins supérieur à 500 GHz,
de sorte que chaque détecteur définit un pixel de résolution de détecteur d'une taille prédéterminée, facultativement une multiplicité d'émetteurs et/ou de détecteurs arrangés dans un groupement linéaire ou bidimensionnel,
lesdits détecteurs étant adaptés pour fournir un signal correspondant à la quantité de SAP pour chacune desdites régions de pixel de ladite structure absorbante;
o un codeur pour générer une séquence d'impulsions servant de signal de référence de temps lié à la vitesse ;
∘ un système de contrôle pour
▪ régler ledit système d'approvisionnement SAP ou
▪ expulsion des structures absorbantes sélectionnées.

8. Procédé de commande mis en oeuvre par ordinateur dans un système de fabrication en continu pour une série de structures absorbantes fonctionnant sur une nappe continue comprenant un matériau SAP, comprenant les étapes consistant à
A - réception dans un système informatique comprenant
- les signaux RF d'un système de détection EM,
dans lequel ledit système de détection comprend un émetteur pour émettre des ondes électromagnétiques dans la étendue de plus de 100, ou plus de 200 GHz et moins de 900 ou moins de 500 GHz,
et
- un signal de déclenchement provenant du système de codeur adapté pour identifier les structures absorbantes individuelles ;
B - générer un profil RF dans le système informatique pour une structure absorbante individuelle dans ledit système informatique ;
C - calculer dans le système informatique un profil de poids SAP réel pour la structure absorbante individuelle ;
D - comparer le profil de poids SAP réel d'une structure absorbante individuelle avec un profil cible prédéfini dans le système informatique ;
E - calculer avec un algorithme prédéfini dans les signaux de commande du système informatique pour les systèmes de dosage et de positionnement SAP ;
F - envoyer le signal de commande de l'étape E) du système informatique aux systèmes de dosage et de positionnement SAP ;
G - actionner les systèmes de dosage et de positionnement SAP par automate(s) à réception des signaux de commande des étapes E et F) ;
H - et répéter les étapes A à G) pour les structures absorbantes suivantes.
